## Europäisches Patentamt
## European Patent Office
## Office européen des brevets

(11) Veröffentlichungsnummer: **0 052 801**
A1

## (12) EUROPÄISCHE PATENTANMELDUNG

(21) Anmeldenummer: 81109211.3

(22) Anmeldetag: 29.10.81

(51) Int. Cl.³: **C 07 C 141/02**
C 07 C 139/14, C 11 D 1/29

(30) Priorität: 26.11.80 DE 3044488

(43) Veröffentlichungstag der Anmeldung:
02.06.82 Patentblatt 82/22

(84) Benannte Vertragsstaaten:
AT BE CH DE FR GB IT LI NL SE

(71) Anmelder: Henkel Kommanditgesellschaft auf Aktien
Postfach 1100 Henkelstrasse 67
D-4000 Düsseldorf 1(DE)

(72) Erfinder: Rupilius, Wolfgang, Dr.
Am Rittersberg 30
D-4000 Düsseldorf 13(DE)

(72) Erfinder: Klötzer, Dietrich, Dr.
Am Rittersberg 8
D-4000 Düsseldorf 13(DE)

(72) Erfinder: Linde, Karl-Heinz
Erlenweg 8
D-4018 Langenfeld(DE)

(54) Verfahren zur Herstellung von Ethersulfaten mit erniedrigtem Dioxangehalt.

(57) Das Verfahren zur Herstellung von Alkali-, Ammonium- und Alkanolammonium-Salzen von Alkylpolyglycolethersulfaten und Alkylarylpolyglycolethersulfaten bezweckt eine Erniedrigung des nach bekannten Verfahren unvermeidlichen Gehaltes an 1.4-Dioxan und umfaßt
a) die Herstellung einer 50 - 80 Gew.-%igen Lösung des Ethersulfats,
b) die Entfernung eines Dioxan-Wasser-Azeotrops durch Verdampfung und
c) gegebenenfalls eine Verdünnung des Ethersulfats auf niedrigere Gebrauchskonzentration.
Die Verdampfung erfolgt vorzugsweise kontinuierlich, in dünner Schicht bei 80° - 95°C und bei einem Druck von 10 - 100 mbar. Durch das neue Verfahren kann der Dioxangehalt beliebig weit, z.B. auf Werte unterhalb 5 ppm gesenkt werden.

EP 0 052 801 A1

HENKEL KGaA
ZR-FE/Patente
Dr. Gl/Ag

4000 Düsseldorf, den 25.11.1980

P a t e n t a n m e l d u n g
D 6284 EP

"Verfahren zur Herstellung von Ethersulfaten mit
erniedrigtem Dioxangehalt"

Gegenstand der Erfindung ist ein Verfahren zur Herstellung
von Alkyl- und Alkylaryl-Polyglycolethersulfaten mit
einem erniedrigten Gehalt an 1.4-Dioxan.

Alkylpolyglycolethersulfate und Alkylarylpolyglycolethersulfate gehören zu den wichtigsten Tensidrohstoffen für
flüssige Spül- und Reinigungsmittel und für kosmetische
Waschmittel und Badepräparate. Größte technische Bedeuttung haben die Natrium-, Ammonium- und Alkanolaminsalze
von Ethersulfaten auf Basis von Anlagerungsprodukten
1 - 4 Mol Ethylenoxid an natürliche und synthetische Fettalkohole mit 10 bis 16 Kohlenstoffatomen. Eine gewisse
technische Bedeutung besitzen auch die Ethersulfate auf
Basis von Anlagerungsprodukten von 2 - 6 Mol Ethylenoxid
an Octhylphenol und Nonylphenol.

Ihre Herstellung erfolgt aus Anlagerungsprodukten des
Ethylenoxids an natürliche und synthetische Fettalkohole,
sowie an Alkylphenole, durch Umsetzung mit Chlorsulfonsäure oder mit Schwefeltrioxid in äquimolaren Mengen.
Dabei werden die Schwefelsäurehalbester der Oxethylate
erhalten, und diese werden dann mit verdünnten wässrigen
Lösungen von Alkalihydroxiden, Ammoniak oder Aminen neutralisiert. Hierbei werden die Neutralisationsbasen in
einer solchen Verdünnung eingesetzt, daß das Ethersulfat
als fließfähige wässrige Lösung anfällt. Die Alkali- und
Ammoniumsalze werden im allgemeinen als 25 - 30 %ige
Lösung hergestellt und in den Handel gebracht, da die
Produkte bei höheren Konzentrationen nicht mehr ausreichend fließfähig sind.

. . .

**0052801**

Bei der Herstellung von Ethersulfaten nach den beschriebenen Verfahren kommt es immer zur Bildung von 1.4-Dioxan als Nebenprodukt, das deshalb in technischen Ethersulfaten, in Abhängigkeit von den Verfahrensbedingungen in Mengen von 300 - 3000 ppm, bezogen auf den Ethersulfat-Gehalt enthalten ist. Dabei zeigen Ethersulfate, die mit $SO_3$ hergestellt wurden, meist Dioxangehalte von über 1000 ppm, solche, die mit $ClSO_3H$ hergestellt wurden, Gehalte von 100 - 500 ppm Dioxan, bezogen auf den Ethersulfat-Gehalt.

Im Jahre 1974 wurden von R.J. Kociba u.a. (Toxicology, Applied Pharmacology Vol. 30, S. 275-298) toxicologische Studien über 1.4-Dioxan veröffentlicht. Obwohl im Tierversuch gesundheitliche Schädigungen erst bei extrem hoher Dosierung beobachtet wurden und eine direkte Gefährdung bei der Verarbeitung und Anwendung von Ethersulfaten nicht wahrscheinlich gemacht werden konnte, besteht seither ein großes Interesse bei Herstellern und Verwendern dieser Tenside, insbesondere im Bereich der kosmetischen Anwender und im Hinblick auf das geschärfte Bewußtsein für toxikologische Fragestellungen, jedes auch nur denkbare Risiko auszuschalten und Ethersulfate, die frei oder weitgehend frei von Dioxan sind, zur Verfügung zu haben.

Die Bildung des 1.4-Dioxans bei der Sulfatierung von Polyglycolethern mit Chlorsulfonsäure oder Schwefeltrioxid kann nicht verhindert werden, es sind auch bisher keine Ansätze zur Lösung dieser Aufgabe bekannt geworden. Die Umsetzung der Polyglycolether mit Amidosulfonsäure liefert zwar weitgehend dioxanfreie Ethersulfate, das Verfahren hat aber wegen der geringeren Umsetzungsgrade und vor allem, da auf diesem Wege nur Ammoniumsalze direkt zugänglich sind, begrenztes technisches Interesse. Die Überführung der so gewonnenen Ammoniumsalze in Alkalisalze mittels Alkalihydroxid ist zwar prinzipiell möglich, jedoch aus wirtschaftlichen Gründen nicht interessant.

...

Eine Abtrennung des 1.4-Dioxans aus den rohen Sulfatierungsprodukten, also aus den Schwefelsäurehalbestern, ist nicht möglich, da diese Produkte nur begrenzt stabil sind und unter thermischer Belastung zur Zersetzung, auch unter Bildung von weiterem 1.4-Dioxan, neigen. Die Abtrennung des 1.4-Dioxans aus den neutralisierten, technischen Ethersulfatlösungen durch Eindampfen oder Wasserdampfdestillation ist im Prinzip möglich, da 1.4-Dioxan mit Wasser bei 1,013 bar ein bei 87,8 $^{o}$C siedendes azeotropes Gemisch aus 81,6 Gewichtsprozent 1.4-Dioxan und 18,4 Gewichtsprozent Wasser bildet. Die handelsüblichen 25 - 30 %igen Lösungen von Ethersulfaten neigen aber unter diesen Bedingungen so sehr zum Schäumen, daß eine destillative Behandlung schon im Labormaßstab schwierig ist und eine großtechnische Durchführung nicht möglich erscheint. Es bestand daher die Aufgabe, ein technisch durchführbares Verfahren zur Abtrennung des 1.4-Dioxans aufzufinden.

Diese Aufgabe wurde erfindungsgemäß und in überraschender Weise gelöst durch ein Verfahren, welches dadurch gekennzeichnet ist, daß

a) zunächst eine Lösung mit einem Gehalt von 50 - 80 Gewichtsprozent des Ethersulfats hergestellt wird,

b) aus diesem konzentrierten Produkt ein Gemisch aus Dioxan und Wasser durch azeotrope Verdampfung entfernt wird und

c) das so behandelte Ethersulfat, gegebenenfalls auf eine niedrigere Gebrauchskonzentration verdünnt wird.

Die für das erfindungsgemäße Verfahren günstigsten Arbeitsbedingungen sind dadurch gekennzeichnet, daß die Verdampfung des Dioxan-Wasser-Gemisches in dünner Schicht bei 80 - 95 $^{o}$C und bei einem Druck von 10 - 100 mbar durchgeführt wird. Die Azeotropdestillation kann aber auch ohne Vakuum und unter Einleitung von ggf. überhitztem Wasserdampf durchgeführt werden.                          ...

Ethersulfate weisen, wie viele andere Tenside, im Bereich einer Konzentration von 50 - 80 Gewichtsprozent eine geringere Zähigkeit auf als in dem mittleren Konzentrationsbereich von 30 - 50 % Aktivsubstanz, der sich meist durch eine gelartige Konsistenz auszeichnet. Die Viskosität in diesem hohen Konzentrationsbereich ist jedoch immer sehr hoch und nur in günstigen Fällen wird bei Raumtemperatur gute Fließfähigkeit bzw. eine Viskosität von weniger als 100 Pas/20° C erreicht. Die exakte Lage des Bereichs verringerter Zähigkeit sowie das dort auftretende Viskositätsminimum sind sowohl von der Struktur des Ethersulfats als auch von Nebenprodukten, z. B. vom Gehalt an Elektrolyten oder an unsulfatiertem Oxethylat abhängig.

Trotz der hohen Viskosität und Tensidkonzentration neigen Ethersulfate im Bereich von 50 - 80 Gewichtsprozent Aktivsubstanz unter den erfindungsgemäßen Bedingungen wesentlich weniger zur Schaumbildung als verdünnte Produkte, so daß überraschenderweise die Abtrennung des Dioxan-Wasser-Azeotrops technisch leicht durchführbar ist. Trotz der bekannten Hydrolyse-Empfindlichkeit der Ethersulfate, besonders in hoher Konzentration und bei hoher Temperatur, zeigt sich unter den erfindungsgemäßen Bedingungen keine Zersetzung oder Hydrolyse, wenn darauf geachtet wird, daß der pH-Wert des Produkts durch einen geringfügigen Überschuß der Neutralisationsbase im alkalischen Bereich gehalten wird. Für die großtechnische Durchführung werden vorteilhaft solche Apparaturen verwendet, die die Verdampfung des Azeotrops kontinuierlich und in dünner Schicht ermöglichen. Daher ist die Verwendung von Dünnschichtverdampfern und Fallfilmverdampfern der Blasendestillation vorzuziehen. Durch die Verdampfung des 1.4-Dioxans

...

gemeinsam mit Wasser kann der Dioxangehalt beliebig weit, gegebenenfalls auch auf Werte unter 5 ppm gesenkt werden, dabei nimmt die Produktkonzentration um so mehr zu, je vollständiger das Dioxan abgetrennt wird. Dieser Konzentrationsanstieg kann durch stufenweise oder kontinuierliche Wasserzugabe oder durch Einblasen von Wasserdampf während der Azeotropdestillation vermieden werden.

Nach der Dioxan-Abtrennung können die Ethersulfate durch Verdünnen mit Wasser nach bekannten Verfahren auf die Konzentration der Handelsware eingestellt werden.

Die nachfolgenden Beispiele sollen den Erfindungsgegenstand erläutern, ohne ihn hierauf zu beschränken:

...

<u>Beispiel 1</u>: Entfernung von 1.4-Dioxan aus einem Fett-
          alkoholethersulfat, Na-Salz (SO₃-Sulfatierung)

Ausgangsprodukt : Lauryl-myristylalkohol (70 : 30) +
                  2 Mol Ethylenoxid

Sulfatierung    : Kontinuierliche Sulfatierung mit
                  SO₃ / Luft in einem Chemithon-Filmreaktor

Neutralisation  : 20 %ige NaOH-Lösung

| | Ethersulfat-Gehalt (MG 384) | 1.4-Dioxan-Gehalt |
|---|---|---|
| Rohprodukt | 65,5 % | 1.470 ppm |
| fortschreitende | 74,6 % | 344 ppm |
| Verdampfung des | 80,2 % | 91 ppm |
| 1.4-Dioxans m.Wasser | 85,1 % | unter 5 ppm |
| bei 90° C/ 50 mbar | (bezogen auf Ge-samtprodukt | (bezogen auf den Ethersulfat-Gehalt |

. . .

<u>Beispiel 2:</u> Entfernung von 1.4-Dioxan aus einem Fett-
alkoholethersulfat, Na-Salz
($ClSO_3H$-Sulfatierung)

Ausgangsprodukt : Lauryl-myristylalkohol (70 : 30) +
2 Mol Ethylenoxid

Sulfatierung    : Kontinuierliche Sulfatierung mit $ClSO_3H$

Neutralisation  : 20 %ige NaOH-Lösung

| | Ethersulfat-Gehalt (MG:384) | 1.4-Dioxan-Gehalt |
|---|---|---|
| Rohprodukt | 68,3 % | 330 ppm |
| fortschreitende Verdampfung des 1.4-Dioxans m.Wasser bei 90° C/ 50 mbar | 76,0 % 82,6 % (bezogen auf Ge-samt-Produkt) | 8 ppm unter 1 ppm (bezogen auf den Ethersulfat-Gehalt) |

. . .

P a t e n t a n s p r ü c h e

1. Verfahren zur Herstellung von Alkali-, Ammonium- und Alkanolammonium-Salzen von Alkylpolyglycolethersulfaten und Alkylarylpolyglycolethersulfaten mit erniedrigtem Gehalt an 1.4.-Dioxan, dadurch gekennzeichnet, daß

a) zunächst eine Lösung mit einem Gehalt von 50 - 80 Gewichtsprozent des Ethersulfats hergestellt wird,

b) aus diesem konzentrierten Produkt ein Gemisch aus Dioxan und Wasser durch azeotrope Verdampfung entfernt wird und

c) das so behandelte Ethersulfat gegebenenfalls auf eine niedrigere Gebrauchskonzentration verdünnt wird.

2. Verfahren nach Anspruch 1, dadurch gekennzeichnet, daß die Verdampfung des Dioxan-Wasser-Gemisches in dünner Schicht bei 80 - 95° C und bei einem Druck von 10 - 100 mbar durchgeführt wird.

## Europäisches Patentamt

## EUROPÄISCHER RECHERCHENBERICHT

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - B - 2 016 656 (HENKEL)<br>* Anspruch 1 *<br>-- | 1 | C 07 C 141/02<br><br>C 07 C 139/14<br><br>C 11 D 1/29 |
| A | DE - B2 - 2 253 896 (THE LION FAT AND OIL CO.)<br>* Anspruch 1 *<br>-- | 1 | |
| A | "Ullmanns Encyklopädie der technischen Chemie"<br>4. Auflage, Band 10, "Dentalchemie bis Erdölverarbeitung"<br>1975, VERLAG CHEMIE, Weinheim/Bergstr.<br>Seiten 151 bis 154<br>* Seite 151, Zeilen 14 bis 16 *<br>----- | 1 | |

### RECHERCHIERTE SACHGEBIETE (Int. Cl.³)

B 01 D 3/36

C 07 C 139/14

C 07 C 141/02

C 07 C 141/14

C 11 D 1/29

### KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 16-02-1982 | KNAACK |

EPA form 1503.1 06.78